# EUROPÄISCHE PATENTSCHRIFT

(11) **EP 2 246 096 B1**
(45) Veröffentlichungstag und Bekanntmachung des Hinweises auf die Patenterteilung: **09.11.2016**
(21) Anmeldenummer: 09159002.6
(22) Anmeldetag: 29.04.2009
(51) Int. Cl.: A61N 5/10

(54) **VERFAHREN UND VORRICHTUNG ZUR BESTIMMUNG BEVORZUGTER AUSRICHTUNGEN EINES BEHANDLUNGSSTRAHLERZEUGERS**
METHOD AND DEVICE FOR DETERMINING PREFERRED ALIGNMENTS OF A RADIATION TREATMENT SOURCE
PROCÉDÉ ET DISPOSITIF DE DÉTERMINATION D'ALIGNEMENTS PRÉFÉRÉS D'UNE SOURCE D'IRRADIATION

(43) Veröffentlichungstag der Anmeldung: 03.11.2010
(73) Patentinhaber: Brainlab AG, 85622 Feldkirchen (DE)
(72) Erfinder: Berlinger, Kajetan, 80798 München (DE); Fröhlich, Stephan, 85609 Aschheim (DE)
(74) Vertreter: Schwabe - Sandmair - Marx

(56) Entgegenhaltungen:
- EP-A- 1 832 313
- US-A- 5 513 238
- US-A1- 2008 310 590
- ROWBOTTOM CARL GRAHAM ET AL: "Improvements in prostate radiotherapy from the customization of beam directions" MEDICAL PHYSICS, AIP, MELVILLE, NY, US, Bd. 25, Nr. 7, 1. Juli 1998 (1998-07-01), Seiten 1171-1179, XP012010526 ISSN: 0094-2405

## Beschreibung

Die vorliegende Erfindung betrifft ein Verfahren und eine Vorrichtung zur Bestimmung bevorzugter Ausrichtungen eines zur Bestrahlung eines Objektes geeigneten Behandlungsstrahlerzeugers gegenüber einem Körper.

In der medizinischen Anwendung ist es bekannt, ein Objekt, das sich in oder an einem Körper befindet, beispielsweise einen Tumor, mit einem Behandlungsstrahl zu bestrahlen. Der Behandlungsstrahl weist üblicherweise Energien im Bereich von Megaelektronenvolt (MeV) auf. Dabei ist es bekannt, die Bestrahlung so zu planen, dass gefährdete Organe (Organs At Risk, OAR) dem Behandlungsstrahl nicht oder möglichst wenig ausgesetzt sind.

Ein Gerät nach dem Stand der Technik wird in EP1832313 beschrieben.

In letzter Zeit geht die Entwicklung dahin, die Position des Objekts während der Bestrahlung in Echtzeit gegenüber der Planung zu verifizieren und den Behandlungsstrahl nachzuführen bzw. ein- oder auszuschalten, wenn sich das Objekt im Strahlengang des Behandlungsstrahls befindet bzw. diesen verlässt. Für die Bestimmung der Position des Objekts während der Bestrahlung werden üblicherweise Röntgeneinrichtungen verwendet, deren relative Lage gegenüber dem Behandlungsstrahlerzeuger bekannt und unveränderlich ist. Der Behandlungsstrahlerzeuger und die Röntgeneinrichtung sind beispielsweise am gleichen Träger angeordnet, der gegenüber dem Körper positionierbar ist.

Es ist die Aufgabe der vorliegenden Erfindung, ein Verfahren und eine Vorrichtung bereit zu stellen, die die Ermittlung der Position des Objekts verbessern.

Diese Aufgabe wird erfindungsgemäß gelöst durch das Verfahren nach Anspruch 1, das Computerprogramm nach Anspruch 9 und die Vorrichtung nach Anspruch 10. Der Anspruch 11 betrifft eine Behandlungsvorrichtung. Vorteilhafte Ausgestaltungsformen sind den abhängigen Ansprüchen zu entnehmen.

Das erfindungsgemäße Verfahren betrifft die Bestimmung bevorzugter Ausrichtungen eines zur Bestrahlung eines Objekts geeigneten Behandlungsstrahlerzeugers gegenüber einem Körper. Erfindungsgemäß wird bei der Bestimmung der bevorzugten Ausrichtungen die Sichtbarkeit des Objekts in mindestens einer Abbildung berücksichtigt. Die Abbildung wird mittels mindestens einer Bildgebungseinrichtung gewonnen, deren relative Lage gegenüber dem Behandlungsstrahlerzeuger bekannt und unveränderlich ist.

Bei der herkömmlichen Planung einer Bestrahlung werden ausschließlich gefährdete Organe berücksichtigt und Ausrichtungen des Behandlungsstrahlerzeugers ausgewählt, bei denen die Strahlenbelastung für gefährdete Organe minimiert wird. Da der Behandlungsstrahlerzeuger und die Bildgebungseinrichtungen, beispielsweise in Form von Röntgeneinrichtungen, eine unveränderliche relative Lage zueinander aufweisen, kann es vorkommen, dass bei der ausgewählten Ausrichtung des Behandlungsstrahlerzeugers das Objekt in den Abbildungen der Bildgebungseinrichtungen von blockierenden Strukturen (Blocking Structures, BS) verdeckt wird. Blockierende Strukturen, wie z. B. Knochen, weisen eine hohe Dichte auf, so dass das Objekt von der Röntgeneinrichtung nicht aufgelöst werden kann. Durch das erfindungsgemäße Verfahren wird bei der Planung der Bestrahlung, also der Bestimmung der bevorzugten Ausrichtung des Behandlungsstrahlerzeugers, die Sichtbarkeit des Objekts in den Abbildungen der Bildgebungseinrichtungen berücksichtigt. Dies führt dazu, dass solche Ausrichtungen als bevorzugt bestimmt werden, bei denen das Objekt in den Abbildungen gut sichtbar ist und somit die Position des Objekts zuverlässig ermittelt werden kann.

Der Begriff Ausrichtung umfasst insbesondere den Einfallswinkel des Behandlungsstrahls auf den Körper in ein oder zwei Freiheitsgraden, kann aber optional auch die translatorische Ausrichtung in ein, zwei oder drei Freiheitsgraden umfassen. Ein rotatorischer Freiheitsgrad ist beispielsweise der Drehwinkel des Behandlungsstrahlerzeugers um eine Drehachse, die beispielsweise die Longitudinalachse des Körpers oder eine dazu parallele Achse ist, wobei der Behandlungsstrahl bevorzugt diese Drehachse schneidet. Der zweite Freiheitsgrad ist beispielsweise ein Kippwinkel um eine Achse, die senkrecht auf dem Behandlungsstrahl steht und bevorzugt die Longitudinalachse rechtwinklig schneidet. Eine Bewegung um die Kippachse kann entweder durch eine Bewegung des Strahlerzeugers oder durch Bewegung einer Auflage, auf der der Körper liegt, bewirkt werden.

Bevorzugt werden bei der Bestimmung der bevorzugten Ausrichtungen gefährdete Organe im Strahlengang des Behandlungsstrahlerzeugers berücksichtigt. Somit fließen in die Planung der Bestrahlung neben der Sichtbarkeit des Objekts auch die gefährdeten Organe ein. Besonders bevorzugt werden diejenigen Ausrichtungen als bevorzugt bestimmt, bei denen der Behandlungsstrahl des Behandlungsstrahlerzeugers möglichst wenige gefährdete Organe trifft und gleichzeitig die größtmögliche Sichtbarkeit des Objekts in den Abbildungen der Bildgebungseinrichtungen gegeben ist. Dabei erfolgt die Bestimmung beispielsweise zweistufig. In einem ersten Schritt wird eine Menge von Ausrichtungen bestimmt, bei denen der Behandlungsstrahl möglichst wenige gefährdete Organe trifft. In einem zweiten Schritt werden aus dieser Menge eine oder mehrere Ausrichtungen bestimmt, in denen die Sichtbarkeit des Objekts in den Abbildungen maximal ist. Dadurch wird der Vermeidung der Bestrahlung gefährdeter Organe höchste Priorität eingeräumt.

Alternativ wird einer Ausrichtung des Behandlungsstrahlerzeugers ein Wert zugeordnet, der sich aus den vom Behandlungsstrahl getroffenen Organen und der Sichtbarkeit des Objekts in den Abbildungen zusammensetzt. Dabei werden die Organe beispielsweise in Risikogruppen gruppiert, wobei ein durch den Behandlungsstrahl stark gefährdetes Organ zu einem niedrigen Wert führt und umgekehrt. Die Gewichtung der Gefährdung der Organe und der Sichtbarkeit des Objekts bei der Bestimmung des Wertes ist dem implementierenden Fachmann überlassen, beispielsweise in Abhängigkeit von der Art des Objekts und/oder des Behandlungsstrahlerzeugers. Insbesondere wird die Ausrichtung mit dem höchsten Wert als bevorzugt bestimmt.

In einer Ausgestaltungsform des Verfahrens wird ein Bilddatensatz, der eine dreidimensionale Abbildung zumindest eines das Objekt enthaltenden Teils des Körpers repräsentiert, bereitgestellt. Die Abbildung wird beispielsweise mittels Computertomographie (CT) oder Magnetresonanztomographie (MRT) aufgenommen. Die Sichtbarkeit des Objekts wird aus einer virtuellen Abbildung bestimmt, die aus dem Bilddatensatz berechnet wird. Die Berechnung einer virtuellen Abbildung, beispielsweise einer Projektion in Form eines DRR (Digitally Reconstructed Radiograph) aus einem dreidimensionalen Bilddatensatz, ist aus dem Stand der Technik bekannt. Der virtuelle Blickwinkel, aus dem die virtuelle Abbildung berechnet wird, entspricht der Blickrichtung der Bildgebungseinrichtung für die zugehörige Ausrichtung des Behandlungsstrahlerzeugers. Da die relative Lage zwischen der oder den Bildgebungseinrichtung(en) und dem Behandlungsstrahlerzeuger bekannt ist, ergibt sich aus der Ausrichtung des Behandlungsstrahlerzeugers automatisch die Blickrichtung der Bildgebungseinrichtung(en).

Bei der Bestimmung der bevorzugten Ausrichtungen werden demnach nicht die tatsächlichen Abbildungen der Bildgebungseinrichtungen berücksichtigt, sondern die virtuellen Abbildungen, die aus dem Bilddatensatz berechnet werden, der im Vorfeld der Bestrahlung gewonnen wurde. Dies erlaubt eine Planung der Bestrahlungswinkel, bevor der Patient in Kontakt mit der Behandlungsvorrichtung gebracht wird.

Werden hierin Daten, Regionen, Bereiche oder Bilder "bereitgestellt", so bedeutet dies, dass sie für die Nutzung durch das erfindungsgemäße Verfahren bereit sind. Diesen Zustand des "Bereitstehens" können die Daten, Abbildungen oder Bilder z.B. durch Erfassen der Daten, Abbildungen oder Bilder (z.B. durch Analysegeräte) oder durch Eingeben der Daten, Abbildungen oder Bilder (z.B. über Schnittstellen) erreichen. Die Daten können diesen Zustand auch dadurch haben, dass sie in einem Speicher (z.B. ROM, CD, Festplatte) abgespeichert und so für eine Verwendung im Rahmen des erfindungsgemäßen Verfahrens bereit stehen. Vor dem Bereitstellen, insbesondere vor dem Abspeichern, können die Daten, Abbildungen oder Bilder auch in einem Schritt des Verfahrens bestimmt, insbesondere berechnet werden.

In einer Fortführung der Erfindung wird die Lage des Objekts aus dem Bilddatensatz und/oder den Abbildungen der Bildgebungseinrichtungen bestimmt. Als Referenzpunkt für die Lage des Objekts dient beispielsweise der Körper, ein Teil einer Einrichtung, beispielsweise der Behandlungsstrahlerzeuger, oder ein beliebiges raumfestes Koordinatensystem. Der Begriff Lage umfasst mindestens die Position und bevorzugt zusätzlich die räumliche Ausrichtung des Objekts.

Die Bestimmung der Lage des Objekts erfolgt beispielsweise mittels Bildfusion (image fusion). Dabei wird der Bilddatensatz so verzerrt und/oder virtuell positioniert, dass aus dem derart modifizierten Bilddatensatz berechnete virtuelle Abbildungen bestmöglich mit den Abbildungen der Bildgebungseinrichtungen übereinstimmen. Die Lage des Objekts ergibt sich dann aus der Lage des Objekts im modifizierten Bilddatensatz. Weitere Methoden zur Ermittlung der Lage eines Objekts anhand der Abbildungen von Bildgebungseinrichtungen sind beispielsweise aus den Europäischen Patentanmeldungen EP 08 169 422.6 und EP 07 150 014.4 der Anmelderin bekannt.

Bevorzugt wird der Behandlungsstrahl des Behandlungsstrahlerzeugers in Abhängigkeit der Lage des Objekts automatisch geschaltet und/oder nachgeführt. Dies ist insbesondere vorteilhaft, wenn sich das Objekt während der Bestrahlung bewegt, beispielsweise aufgrund der Atmung eines Patienten. Wird der Behandlungsstrahl geschaltet, so bedeutet dies, dass der Behandlungsstrahl eingeschaltet wird, wenn sich das Objekt im Strahlengang befindet, und der Behandlungsstrahl ausgeschaltet wird, wenn das Objekt nicht im Strahlengang des Behandlungsstrahls liegt. Dies wird auch als gating bezeichnet. Wird der Behandlungsstrahl nachgeführt, so bedeutet dies, dass der Behandlungsstrahl der sich verändernden Lage des Objekts folgt. Dies wird auch als tracking bezeichnet.

In einer Ausgestaltungsform der Erfindung enthält der Bilddatensatz mehrere dreidimensionale Abbildungen zumindest des das Objekt enthaltenden Teils des Körpers zu verschiedenen Zeitpunkten, beispielsweise verschiedenen Stadien des Atemzyklus des Patienten. Ein solcher Bilddatensatz wird auch als 4D-Bilddatensatz bezeichnet. Aus den Abbildungen der Bildgebungseinrichtungen wird beispielsweise diejenige dreidimensionale Abbildung ausgewählt, deren virtuelle Projektion (DRR) die größte Übereinstimmung mit den Abbildungen der Bildgebungseinrichtungen aufweist. Aus dieser dreidimensionalen Abbildung kann dann die Lage des Objekts bestimmt werden. Dazu ist die dreidimensionale Abbildung bevorzugt segmentiert, so dass die Voxel des Bilddatensatzes verschiedenen Strukturen, unter anderem dem Objekt, zugeordnet sind.

Bevorzugt wird bei der Nutzung eines 4D-Bilddatensatzes ein kumulatives Maß für die Sichtbarkeit über mehrere oder alle dreidimensionalen Abbildungen im 4D-Bilddatensatz verwendet. Dazu werden für eine untersuchte Ausrichtung die Sichtbarkeiten des Objekts in den einzelnen dreidimensionalen Abbildungen bestimmt und aus den einzelnen Sichtbarkeiten die kumulierte Sichtbarkeit ermittelt. Die kumulierte Sichtbarkeit ist beispielsweise die Summe der Sichtbarkeiten, der gewichtete oder ungewichtete Mittelwert über die Sichtbarkeiten oder die geringste Sichtbarkeit in den dreidimensionalen Abbildungen. Alternativ werden diejenigen dreidimensionalen Abbildungen im 4D-Bilddatensatz bestimmt, in denen die Sichtbarkeit des Objekts unter einem Schwellenwert liegt. Beispielsweise können nur diejenigen Ausrichtungen als bevorzugt bestimmt werden, für die die Sichtbarkeit in einer Mindestanzahl von dreidimensionalen Abbildungen im 4D-Bilddatensatz über dem Schwellenwert liegt. Optional wird der Behandlungsstrahl dem Objekt nachgeführt, jedoch ausgeschaltet, wenn die Sichtbarkeit des Objekts unter einem Schwellenwert liegt, da die Lage des Objekts in diesem Fall nicht sicher bestimmt werden kann. Dies entspricht einer Kombination aus tracking und gating.

In einer Ausgestaltungsform der Erfindung wird eine Menge bevorzugter Ausrichtungen bestimmt und die Menge einem Benutzer zur Auswahl einer oder mehrerer Ausrichtungen dargestellt. Es erfolgt somit eine automatische Vorauswahl bevorzugter Ausrichtungen, aus denen der Benutzer eine oder mehrere für die spätere Bestrahlung auswählen kann. Beispielsweise wird dem Benutzer für jede bevorzugte Ausrichtung aus der Menge die Sichtbarkeit des Objekts quantitativ oder qualitativ angegeben, beispielsweise in Prozent. Alternativ oder zusätzlich wird die virtuelle Abbildung, also das Projektionsbild (DRR), die der Abbildung einer Bildgebungseinrichtung für die zugehörige Ausrichtung des Behandlungsstrahlerzeugers entspricht, dargestellt. Weiterhin alternativ oder zusätzlich wird der Behandlungsstrahlerzeuger in jede der Ausrichtungen gebracht, mittels jeder der Bildgebungseinrichtung eine Abbildung erzeugt und diese Abbildung dargestellt. Somit kann der Benutzer die tatsächliche Sichtbarkeit des Objekts in den Abbildungen der Bildgebungseinrichtungen für jede Ausrichtung aus der Menge beurteilen. Vorteilhaft wird das Objekt in virtuellen Abbildungen farblich hervorgehoben.

In einer weiteren Ausgestaltungsform der Erfindung wird automatisch eine optimale Ausrichtung aus den als bevorzugt bestimmten Ausrichtungen ausgewählt. Beispielsweise wird diejenige Ausrichtung ausgewählt, die zur maximalen Sichtbarkeit des Objekts in den Abbildungen der Bildgebungseinrichtungen führt. Sind mehrere Bildgebungseinrichtungen vorgesehen, so dient als Kriterium die größte Sichtbarkeit aus allen Abbildungen, ein gewichteter oder ungewichteter Mittelwert über die Sichtbarkeiten in allen Abbildungen oder die Sichtbarkeit in der Abbildung mit der geringsten Sichtbarkeit des Objekts. Bevorzugt wird die ausgewählte Ausrichtung dem Benutzer dargestellt, beispielsweise zusammen mit der quantitativen Angabe der Sichtbarkeit, der virtuellen Abbildung für eine zu der ausgewählten Ausrichtung korrespondierenden Blickrichtung oder den Abbildungen der Bildgebungseinrichtungen für die ausgewählte Ausrichtung.

In vorteilhafter Weise ist die Sichtbarkeit des Objekts definiert als der in einer (realen oder virtuellen) Abbildung sichtbare Teil des Objekts bezogen auf die Gesamtgröße des Objekts. Der sichtbare Teil und die Gesamtgröße können zweidimensional sein, also beispielsweise zu der Fläche des Objekts in einer zweidimensionalen Abbildung korrespondieren, oder dreidimensional sein, sich also auf das Volumen des Objekts beziehen. Dabei wird der Datensatz mit der dreidimensionalen Abbildung bevorzugt segmentiert, um die Kontur des Objekts zu erhalten. Diese Kontur wird beispielsweise auf das DRR projiziert. Die Konturen des (segmentierten) Objekts in der dreidimensionalen Abbildung und des im DRR-Projektionsbild sichtbaren Objekts werden miteinander abgeglichen und daraus eine prozentuale Übereinstimmung berechnet, die die Sichtbarkeit quantifiziert.

Das erfindungsgemäße Verfahren ist insbesondere ein Datenverarbeitungsverfahren. Das Datenverarbeitungsverfahren wird bevorzugt durch technische Mittel, insbesondere einen Computer, durchgeführt. Der Computer umfasst insbesondere einen Prozessor und insbesondere einen Speicher, um insbesondere elektronisch die Daten zu verarbeiten. Insbesondere werden die beschriebenen Schritte des Berechnens oder Bestimmens von einem Computer im Rahmen des technischen Datenverarbeitungsverfahrens ausgeführt.

Die Erfindung betrifft weiterhin ein Computerprogramm, das, wenn es in einer Recheneinheit ausgeführt wird, die Recheneinheit dazu veranlasst, das vorstehend beschriebene Verfahren auszuführen.

Im Rahmen der Erfindung können Computerprogrammelemente von Hardware und/oder Software (dies beinhaltet auch Firmware, im System abgespeicherte Software, Mikrocode usw.) verkörpert werden. Im Rahmen der Erfindung können Computerprogrammelemente die Form eines Computerprogrammprodukts annehmen, das durch ein auf einem Computer verwendbares oder computerlesbares Speichermedium verkörpert werden kann, das auf einem Computer verwendbare oder computerlesbare Programmanweisungen, "Code" oder ein "Computerprogramm" umfasst, die bzw. der bzw. das auf dem genannten Medium zur Verwendung auf oder in Verbindung mit dem System, das die Anweisung ausführt, verkörpert sind bzw. ist. Ein solches System kann ein Computer sein, ein Computer kann eine Datenverarbeitungsvorrichtung mit Mitteln zum Ausführen der Computerprogrammelemente und/oder des erfindungsgemäßen Programms sein. Im Rahmen dieser Erfindung kann ein auf einem Computer verwendbares oder computerlesbares Medium irgendein Medium sein, dass das Programm zur Verwendung auf oder in Verbindung mit dem System, Apparat oder der Einrichtung, das bzw. der bzw. die die Anweisung ausführt, das Programm beinhalten, speichern, übermitteln, fortpflanzen oder transportieren kann. Das auf einem Computer verwendbare oder computerlesbare Medium kann z.B. ein elektronisches, magnetisches, optisches, elektromagnetisches, Infrarot- oder Halbleiter-System, ein solcher Apparat oder eine solche Einrichtung oder ein Ausbreitungsmedium wie z.B. das Internet sein, ist aber nicht auf diese Aufzählung beschränkt. Das auf einem Computer verwendbare oder computerlesbare Medium könnte sogar z.B. Papier oder ein anderes geeignetes Medium sein, auf das das Programm gedruckt ist, da das Programm elektronisch erfasst werden könnte durch z.B. optisches Scannen des Papiers oder anderen geeigneten Mediums und dann kompiliert, interpretiert oder andernfalls auf geeignete Weise prozessiert werden könnte. Das Computerprogrammprodukt und jegliche Software und/oder Hardware, das bzw. die hier beschrieben werden, bilden in den beispielhaften Ausführungsformen die verschiedenen Mittel zum Ausführen der Funktionen der Erfindung.

Die Erfindung betrifft weiterhin eine Vorrichtung zur Bestimmung bevorzugter Ausrichtungen eines zur Bestrahlung eines Objekts geeigneten Behandlungsstrahlerzeugers gegenüber einem Körper. Die Vorrichtung weist eine Recheneinheit auf, die dazu ausgebildet ist, bei der Bestimmung der bevorzugten Ausrichtungen die Sichtbarkeit des Objekts in mindestens einer Abbildung zu berücksichtigen. Die Abbildung wird mittels mindestens einer Bildgebungseinrichtung gewonnen, deren relative Lage gegenüber dem Behandlungsstrahlerzeuger bekannt und unveränderlich ist. Die Vorrichtung bzw. Komponenten der Vorrichtung wie die Recheneinheit sind demnach dazu ausgebildet, das vorstehend beschriebene Verfahren auszuführen.

Die Erfindung betrifft weiterhin eine Behandlungsvorrichtung mit einem Behandlungsstrahlerzeuger, mindestens einer Bildgebungseinrichtung, deren relative Lage zum Behandlungsstrahlerzeuger bekannt und unveränderlich ist, und einer Vorrichtung zur Bestimmung bevorzugter Ausrichtungen. Bevorzugt sind der Behandlungsstrahlerzeuger und die Bildgebungseinrichtung(en) auf einem gemeinsamen Träger angeordnet. Weiterhin bevorzugt ist ein Antrieb zur Ausrichtung des Trägers gegenüber dem Körper vorgesehen. Somit kann der Behandlungsstrahlerzeuger in die vom Benutzer oder automatisch ausgewählte Ausrichtung gebracht werden.

Die Vorrichtung weist bevorzugt eine Schnittstelle auf, über die ihr ein Bilddatensatz bereitgestellt wird, der mindestens eine dreidimensionale Abbildung zumindest eines das Objekt enthaltenden Teils des Körpers repräsentiert. Die Bereitstellung kann beispielsweise durch einen internen oder externen Speicher, ein Netzwerk oder direkt von einer Bilddatensatzerzeugungsvorrichtung, wie einem Computertomographen oder Magnetresonanztomographen, erfolgen.

Bei der Bildgebungseinrichtung handelt es sich bevorzugt um eine Röntgeneinrichtung. Eine solche Röntgeneinrichtung erzeugt üblicherweise Röntgenstrahlung im Energiebereich von Kiloelektronenvolt (keV). Der Röntgenstrahl ist beispielsweise kegelförmig. Hinter dem Körper trifft der Röntgenstrahl auf einen Detektor, der beispielsweise zweidimensional ausgebildet ist und ein Röntgen- Projektionsbild erfasst.

Die Vorrichtung weist weiterhin bevorzugt eine Anzeigevorrichtung zur Anzeige von vorgeschlagenen oder ausgewählten bevorzugten Einrichtungen auf. Verschiedene Möglichkeiten zur Anzeige der bevorzugten Ausrichtungen wurden bereits im Rahmen des Verfahrens geschildert.

In einer Ausgestaltungsform der Erfindung sind zwei Bildgebungseinrichtungen, beispielsweise zwei Röntgeneinrichtungen, vorgesehen. Dies ermöglicht eine zuverlässigere Detektion der Position des Objekts, insbesondere wenn sich das Objekt entlang der Mittelachse der Röntgenstrahlung einer der Röntgeneinrichtungen bewegt. Darüber hinaus besteht eine größere Wahrscheinlichkeit dafür, dass zumindest eine der Bildgebungseinrichtungen so ausgerichtet ist, dass sich eine ausreichende Sichtbarkeit des Objekts in der von ihr erzeugten Abbildung ergibt.

Alternativ oder zusätzlich ist ein weiterer Sensor vorgesehen, der so ausgebildet und angeordnet ist, dass der Behandlungsstrahl nach der Durchstrahlung des Körpers auf den Sensor trifft und dadurch eine (zusätzliche) Abbildung des Körpers erzeugt wird. Die Kombination aus Handlungsstrahlerzeuger und zusätzlichem Sensor dient somit als Bildgebungseinrichtung.

Es ist im Rahmen der Erfindung möglich, nicht zur Ausführbarkeit der Erfindung notwendige Merkmale wegzulassen oder die vorstehend geschilderten Merkmale beliebig zu kombinieren.

Die vorliegende Erfindung soll anhand eines Ausführungsbeispiels näher erläutert werden.

Dabei zeigt
- Figur 1: schematisch eine erfindungsgemäße Behandlungsvorrichtung,
- Figur 2a: eine erste virtuelle Abbildung und
- Figur 2b: eine zweite virtuelle Abbildung.

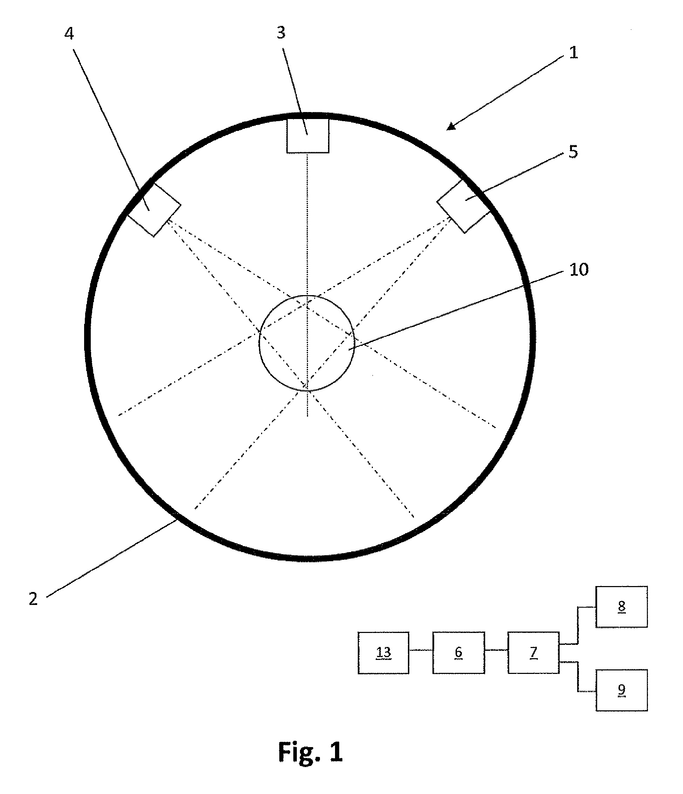

Die Figur 1 zeigt schematisch eine Behandlungsvorrichtung 1 mit einem kreisförmigen Träger 2, an dem ein Behandlungsstrahlerzeuger 3 und zwei Röntgeneinrichtungen 4 und 5 angeordnet sind. Aus Gründen der Übersichtlichkeit zeigt Figur 1 nur die Röntgenquellen, nicht jedoch die zugehörigen Röntgendetektoren. Die Röntgenquellen der Röntgeneinrichtungen 4 und 5 sind beidseitig um 45 Grad gegenüber dem Behandlungsstrahlerzeuger 3 versetzt angeordnet. Der Abstand der Röntgeneinrichtungen vom Behandlungsstrahlerzeuger kann auch anders gewählt werden, beispielsweise zwischen 0 und 90 Grad, insbesondere von 45 Grad bis 90 Grad. Der Träger 2 kann neben der beispielhaft dargestellten Form eines Kreises auch die Form beispielsweise eines Kreisbogens aufweisen.

Eine Recheneinheit 6 ist über nicht dargestellte Leitungen mit dem Behandlungsstrahlerzeuger 3, den Röntgeneinrichtungen 4 und 5 sowie einer nicht dargestellten Antriebseinheit für den Träger 2 verbunden. Die Antriebseinheit ist in der Lage, den Träger 2 und die daran angeordneten Komponenten um eine Längsachse zu drehen, die im vorliegenden Beispiel senkrecht auf der Papierebene steht und durch den Mittelpunkt des kreisförmigen Trägers 2 verläuft. Optional ist der Träger 2 um eine Kippachse kippbar, die die Längsachse im rechten Winkel schneidet und in der vorliegenden Darstellung horizontal in der Papierebene liegt.

Die Röntgenquellen der Röntgeneinrichtungen 4 und 5 erzeugen kegelförmige Röntgenstrahlen, die in Figur 1 durch strichpunktierte Linien angedeutet sind. Die Mittelachse des kegelförmigen Röntgenstrahls wird auch als Blickrichtung der Röntgeneinrichtung bezeichnet. Der Behandlungsstrahlerzeuger erzeugt im vorliegenden Ausführungsbeispiel einen punktiert dargestellten Nadelstrahl im MeV-Bereich, kann jedoch je nach Ausgestaltungsform auch einen kegelförmigen Strahl erzeugen.

Der Behandlungsstrahl des Behandlungsstrahlerzeugers 3 dient der Bestrahlung eines Objekts, beispielsweise eines Tumors, in oder an einem Körper 10. Die Röntgeneinrichtungen 4 und 5 erzeugen Abbildungen in Form von Röntgenbildern des Körpers 10. Die relative Lage der Röntgeneinrichtungen 4 und 5 gegenüber dem Behandlungsstrahlerzeuger 3 ist unveränderlich und bekannt.

Die Recheneinheit 6 ist weiterhin mit einem Interface 7 verbunden. Über das Interface 7 wird der Recheneinheit 6 ein Bilddatensatz bereitgestellt, der eine dreidimensionale Abbildung zumindest eines das Objekt enthaltenden Teils des Körpers 10 repräsentiert. Der Bilddatensatz ist beispielsweise in einem Speicher 8 hinterlegt und wird über das Interface 7 an die Recheneinheit 6 übertragen. Alternativ oder zusätzlich ist die Recheneinheit 6 über das Interface 7 mit einem Magnetresonanztomographen oder Computertomographen 9 verbunden, der den Bilddatensatz aufnimmt und bereitstellt.

Im Vorfeld der Bestrahlung des Körpers 10 mit dem Behandlungsstrahl sind eine oder mehrere Ausrichtungen des Behandlungsstrahlerzeugers 3 gegenüber dem Körper 10 festzulegen. Dies wird auch als Planung bezeichnet. Dabei ist insbesondere darauf zu achten, dass der Behandlungsstrahl möglichst wenige gefährdete Organe trifft, um eine Schädigung dieser Organe zu vermeiden. Während der Bestrahlung wird die Position des Objekts ermittelt, um feststellen zu können ob der Behandlungsstrahl das Objekt trifft. Dazu werden mittels der Röntgeneinrichtungen 4 und 5 Röntgenabbildungen des Körpers 10 gewonnen. Die Ermittlung der Position eines Objekts anhand der Röntgenabbildungen ist aus dem Stand der Technik bekannt, beispielsweise aus den Europäischen Patentanmeldungen EP 08 169 422.6 und EP 07 150 014.4 der Anmelderin. Die Vorgehensweise bei der Bestimmung der Position des Objekts aus den Abbildungen wird daher nicht weiter ausgeführt.

Die Ermittlung der Position des Objekts erfolgt insbesondere wiederholt, um beispielsweise eine Verschiebung des Objekts aufgrund einer Atembewegung des Patienten verfolgen zu können.

Um die Position des Objekts aus den Röntgenabbildungen bestimmen zu können, ist es vorteilhaft, dass ein möglichst großer Teil des Objekts in den Röntgenabbildungen sichtbar ist. Dies ist beispielsweise dann nicht der Fall, wenn das Objekt von dichten Strukturen wie beispielsweise Knochen verdeckt wird. Erfindungsgemäß wird daher bei der Bestimmung der Ausrichtung des Behandlungsstrahlerzeugers 3 gegenüber dem Körper 10 nicht nur auf das Kriterium der gefährdeten Organe abgestellt, sondern auch auf die Sichtbarkeit des Objekts in den Röntgenabbildungen der Röntgeneinrichtungen 4 und 5. Da die Lage der Röntgeneinrichtungen 4 und 5 gegenüber dem Behandlungsstrahlerzeuger 3 unveränderlich ist, sind die Blickrichtungen der Röntgeneinrichtungen 4 und 5 bei einer gegebenen Ausrichtung des Behandlungsstrahlerzeugers 3 bekannt. In der Planungsphase kann daher für eine Ausrichtung des Behandlungsstrahlerzeugers 3 aus dem dreidimensionalen Bilddatensatz des Körpers 10 eine virtuelle Abbildung berechnet werden, die zu der Röntgenabbildung einer Röntgeneinrichtung 4 oder 5 korrespondiert. Die Berechnung einer virtuellen Abbildung, die auch als DRR bezeichnet wird, aus einem dreidimensionalen Bilddatensatz ist im Stand der Technik bekannt und wird daher an dieser Stelle nicht detailliert beschrieben.

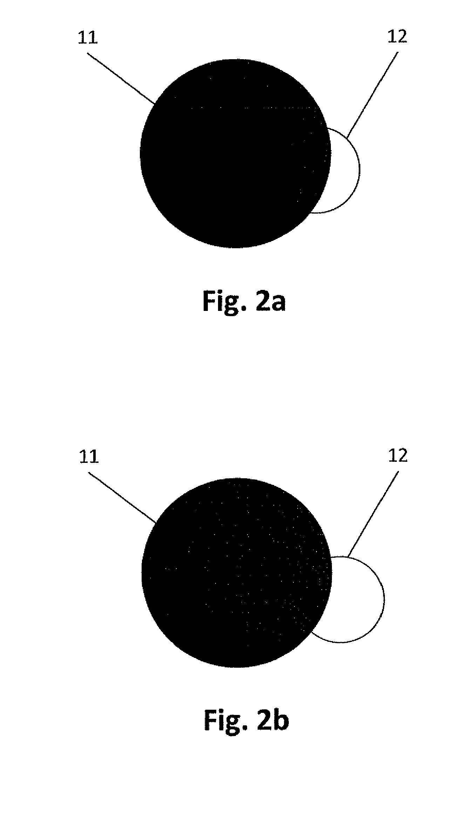

Die Figuren 2a und 2b zeigen schematisch einen Ausschnitt aus einer Röntgenabbildung aus zwei verschiedenen Blickrichtungen. Das Bezugszeichen 11 zeichnet eine blockierende Struktur wie einen Knochen, das Bezugszeichen 12 bezeichnet das zu bestrahlende Objekt. Bei der Blickrichtung, die der Figur 2a zugrunde liegt, sind ca. 30% des Objekts 12 sichtbar, während es bei der Darstellung in der Figur 2b ungefähr 60% sind. Die Sichtbarkeit bezieht sich im vorliegenden Ausführungsbeispiel auf den Anteil der sichtbaren Fläche des Objekts 12 im Röntgenbild. Sind die Ausrichtungen des Behandlungsstrahlerzeugers 3, die zu den Blickrichtungen in den Figuren 2a und 2b führen, bezüglich der gefährdeten Organe gleich geeignet, so wird vorteilhaft die Ausrichtung gewählt, die zu der Blickrichtung der Figur 2b führt, da bei dieser Ausrichtung ein größerer Teil des Objekts 12 sichtbar ist und somit die Positionsbestimmung des Objekts 12 zuverlässiger ist als bei der Blickrichtung, die der Figur 2a zugrunde liegt.

Die Ausführungen zu den Blickrichtungen anhand der Figuren 2a und 2b beziehen sich auf eine einzelne der Röntgeneinrichtungen 4 oder 5. Werden beide Röntgeneinrichtungen berücksichtigt, so bestehen mehrere Möglichkeiten der Beurteilung der Sichtbarkeit. In einer ersten Variante wird diejenige Ausrichtung des Behandlungsstrahlerzeugers 3 gewählt, die zu einer Blickrichtung einer Röntgeneinrichtung führt, die insgesamt die größte Sichtbarkeit des Objekts 12 in allen Abbildungen erzielt. In einer zweiten Variante werden die Sichtbarkeiten des Objekts 12 in den Abbildungen für jede Ausrichtung des Behandlungsstrahlerzeugers 3 gemittelt und diejenige Ausrichtung gewählt, die zur größten mittleren Sichtbarkeit führt. In einer dritten Variante wird für jede Ausrichtung des Behandlungsstrahlerzeugers 3 die geringste Sichtbarkeit des Objekts 12 in allen Abbildungen ermittelt und die Ausrichtung mit der größten minimalen Sichtbarkeit gewählt.

Die Recheneinheit 6 ist weiterhin mit einer Anzeigeeinrichtung 13 verbunden. Die Recheneinheit 6 ist beispielsweise dazu eingerichtet, auf der Anzeigeeinrichtung 13 die virtuelle Abbildung oder die virtuellen Abbildungen darzustellen, die sich aus einer oder mehreren automatisch ausgewählten Ausrichtungen des Behandlungsstrahlerzeugers 3 ergeben. Dies erlaubt einem Benutzer der Behandlungsvorrichtung 1 eine Kontrolle der ausgewählten Ausrichtungen.

Alternativ oder zusätzlich ist die Recheneinheit dazu eingerichtet, eine Menge möglicher Ausrichtungen des Behandlungsstrahlerzeugers 3 zu erzeugen und auf der Anzeigevorrichtung 13 auszugeben, beispielsweise in Form der zu der jeweiligen Ausrichtung korrespondierenden virtuellen Abbildung(en).

Optional werden die virtuellen Abbildungen durch die Bildgebungseinrichtungen, im vorliegenden Ausführungsbeispiel durch die Röntgeneinrichtungen 4 und 5, verifiziert. Dazu wird der Behandlungsstrahlerzeuger 3 entsprechend der Ausrichtung ausgerichtet und es werden mittels der Röntgeneinrichtungen 4 und 5 Röntgenbilder erzeugt. Diese tatsächlichen Röntgenbilder werden dann mit den virtuellen Abbildungen, die für diese Ausrichtung aus dem dreidimensionalen Bilddatensatz berechnet wurden, verglichen. Dazu werden die tatsächlichen Röntgenabbildungen und optional die zugehörigen virtuellen Abbildungen beispielsweise auf der Anzeigevorrichtung 13 angezeigt.

Zur Begrenzung des Rechenaufwandes bei der Bestimmung der bevorzugten Ausrichtungen des Behandlungsstrahlerzeugers wird die Anzahl der untersuchten Ausrichtungen bevorzugt begrenzt, beispielsweise auf 50, 100, 200, 500 oder 1000 Ausrichtungen.

Das vorstehend geschilderte Ausführungsbeispiel ist rein exemplarisch und insofern nicht als beschränkend zu verstehen. Insbesondere können mehr oder weniger als zwei Röntgeneinrichtungen vorgesehen sein. Alternativ oder zusätzlich kann ein Sensor vorhanden sein, der den Behandlungsstrahl nach dem Durchstrahlen des Körpers detektiert und somit zusammen mit dem Behandlungsstrahlerzeuger eine Bildgebungseinrichtung darstellt.

## Patentansprüche

1. Verfahren zur Bestimmung bevorzugter Ausrichtungen eines zur Bestrahlung eines Objekts (12) geeigneten Behandlungsstrahlerzeugers (3) gegenüber einem Körper (10), wobei das Verfahren ein Datenverarbeitungsverfahren ist, das von einem Computer ausgeführt wird, **dadurch gekennzeichnet, dass** bei der Bestimmung der bevorzugten Ausrichtungen die Sichtbarkeit des Objekts (12) in mindestens einer Abbildung berücksichtigt wird, wobei die Abbildung mittels mindestens einer Bildgebungseinrichtung (4, 5), deren relative Lage gegenüber dem Behandlungsstrahlerzeuger (3) bekannt und unveränderlich ist, gewonnen wird.

2. Verfahren nach Anspruch 1, **dadurch gekennzeichnet, dass** bei der Bestimmung der bevorzugten Ausrichtungen gefährdete Organe im Strahlengang des Behandlungsstrahlerzeugers (3) berücksichtigt werden.

3. Verfahren nach Anspruch 2, **dadurch gekennzeichnet, dass** diejenigen Ausrichtungen als bevorzugt bestimmt werden, bei denen der Behandlungsstrahl des Behandlungsstrahlerzeugers (3) möglichst wenige gefährdete Organe trifft und gleichzeitig die größtmögliche Sichtbarkeit des Objekts (12) in den Abbildungen der Bildgebungseinrichtungen gegeben ist.

4. Verfahren nach einem der Ansprüche 1 bis 3, **dadurch gekennzeichnet, dass** ein Bilddatensatz, der eine dreidimensionale Abbildung zumindest eines das Objekt (12) enthaltenden Teils des Körpers (10) repräsentiert, bereitgestellt wird und die Sichtbarkeit des Objekts (12) aus einer virtuellen Abbildung, die aus dem Bilddatensatz berechnet wird, bestimmt wird.

5. Verfahren nach Anspruch 4, **dadurch gekennzeichnet, dass** die Lage des Objekts (12) aus dem Bilddatensatz und den Abbildungen der Bildgebungseinrichtungen (4, 5) bestimmt wird.

6. Verfahren nach einem der Ansprüche 1 bis 5, **dadurch gekennzeichnet, dass** eine Menge bevorzugter Ausrichtungen bestimmt wird und die Menge einem Benutzer zur Auswahl einer oder mehrerer Ausrichtungen dargestellt wird.

7. Verfahren nach einem der Ansprüche 1 bis 5, **dadurch gekennzeichnet, dass** automatisch die optimale Ausrichtung aus den als bevorzugt bestimmten Ausrichtungen ausgewählt wird.

8. Verfahren nach einem der Ansprüche 1 bis 7, **dadurch gekennzeichnet, dass** die Sichtbarkeit des Objekts (12) definiert ist als der in einer Abbildung sichtbare Teil des Objekts (12) bezogen auf die Gesamtgröße des Objekts (12).

9. Computerprogramm, das, wenn es in einer Recheneinheit (6) ausgeführt wird, die Recheneinheit (6) dazu veranlasst, das Verfahren nach einem der Ansprüche 1 bis 8 auszuführen.

10. Vorrichtung zur Bestimmung bevorzugter Ausrichtungen eines zur Bestrahlung eines Objekts geeigneten Behandlungsstrahlerzeugers (3) gegenüber einem Körper (10), **gekennzeichnet durch** eine Recheneinheit (6), die dazu ausgebildet ist, bei der Bestimmung der bevorzugten Ausrichtungen die Sichtbarkeit des Objekts (12) in mindestens einer Abbildung zu berücksichtigen, wobei die Abbildung mittels mindestens einer Bildgebungseinrichtung (4, 5), deren relative Lage gegenüber dem Behandlungsstrahlerzeuger (3) bekannt und unveränderlich ist, gewonnen wird.

11. Behandlungsvorrichtung (1) mit einem Behandlungsstrahlerzeuger (3), mindestens einer Bildgebungseinrichtung (4, 5), deren relative Lage zum Behandlungsstrahlerzeuger (3) bekannt und unveränderlich ist, und einer Vorrichtung nach Anspruch 10.

12. Behandlungsvorrichtung (1) nach Anspruch 11, **gekennzeichnet durch** einen Träger (2), auf dem der Behandlungsstrahlerzeuger (3) und die Bildgebungseinrichtungen (4, 5) angeordnet sind, und einen Antrieb zur Ausrichtung des Trägers (2) gegenüber dem Körper (10).

13. Vorrichtung (1) nach einem der Ansprüche 11 bis 12, **dadurch gekennzeichnet, dass** die Bildgebungseinrichtung (4, 5) eine Röntgeneinrichtung ist.

14. Vorrichtung (1) nach einem der Ansprüche 11 bis 13, **gekennzeichnet durch** eine Anzeigevorrichtung (13) zur Anzeige von vorgeschlagenen oder ausgewählten bevorzugten Ausrichtungen.

## Claims

1. A method for determining preferred alignments of a treatment beam generator (3), which is suitable for irradiating an object (12), relative to a body (10), wherein the method is a data processing method which is performed by a computer, **characterised in that** the visibility of the object (12) in at least one image is taken into account when determining the preferred alignments, wherein the image is obtained by means of at least one imaging device (4, 5), the position of which relative to the treatment beam generator (3) is known and invariable.

2. The method according to Claim 1, **characterised in that** organs at risk in the beam path of the treatment beam generator (3) are taken into account when determining the preferred alignments.

3. The method according to Claim 2, **characterised in that** the alignments which are determined as preferred alignments are those at which the treatment beam of the treatment beam generator (3) hits as few organs at risk as possible and at which the greatest possible visibility of the object (12) in the images of the imaging devices is simultaneously obtained.

4. The method according to any one of Claims 1 to 3, **characterised in that** an image data set is provided which represents a three-dimensional image of at least a part of the body (10) containing the object (12), and the visibility of the object (12) is determined from a virtual image which is calculated from the image data set.

5. The method according to Claim 4, **characterised in that** the position of the object (12) is determined from the image data set and the images of the imaging devices (4, 5).

6. The method according to any one of Claims 1 to 5, **characterised in that** a number of preferred alignments are determined and displayed to a user in order for one or more alignments to be selected.

7. The method according to any one of Claims 1 to 5, **characterised in that** the optimum alignment is automatically selected from the alignments determined as preferred alignments.

8. The method according to any one of Claims 1 to 7, **characterised in that** the visibility of the object (12) is defined as the part of the object (12) which is visible in an image, in relation to the overall size of the object (12).

9. A computer program which, when it is executed in a computational unit (6), causes the computational unit (6) to perform the method according to any one of Claims 1 to 8.

10. A device for determining preferred alignments of a treatment beam generator (3), which is suitable for irradiating an object, relative to a body (10), **characterised by** a computational unit (6) which is designed to take into account the visibility of the object (12) in at least one image when determining the preferred alignments, wherein the image is obtained by means of at least one imaging device (4, 5), the position of which relative to the treatment beam generator (3) is known and invariable.

11. A treatment device (1), comprising: a treatment beam generator (3); at least one imaging device (4, 5), the position of which relative to the treatment beam generator (3) is known and invariable; and a device according to Claim 10.

12. The treatment device (1) according to Claim 11, **characterised by** a support (2) on which the treatment beam generator (3) and the imaging devices (4, 5) are arranged, and a drive for aligning the support (2) relative to the body (10).

13. The device (1) according to any one of Claims 11 to 12, **characterised in that** the imaging device (4; 5) is an X-ray device.

14. The device (1) according to any one of Claims 11 to 13, **characterised by** an indicating device (13) for indicating proposed or selected preferred alignments.

## Revendications

1. Procédé pour déterminer des alignements préférés d'un générateur de faisceau de traitement (3) par rapport à un corps (10), lequel générateur de faisceau de traitement est adapté pour soumettre un objet (12) à un rayonnement, le procédé étant un procédé de traitement de données qui est mis en oeuvre par un ordinateur, **caractérisé en ce que** lors de la détermination des alignements préférés, la visibilité de l'objet (12) est prise en compte dans au moins une image, dans lequel l'image est obtenue au moyen d'au moins un dispositif d'imagerie (4, 5) dont la position relative par rapport au générateur de faisceau de traitement (3) est connue et invariable.

2. Procédé selon la revendication 1, **caractérisé en ce que** lors de la détermination des alignements préférés, des organes à risque sont pris en compte dans le trajet de faisceau du générateur de faisceau de traitement (3).

3. Procédé selon la revendication 2, **caractérisé en ce que** les alignements déterminés comme préférés sont les alignements dans lesquels le faisceau de traitement du générateur de faisceau de traitement (3) rencontre le plus petit nombre possible d'organes à risque et donne simultanément la plus grande visibilité possible de l'objet (12) dans les images des dispositifs d'imagerie.

4. Procédé selon l'une des revendications 1 à 3, **caractérisé en ce qu'**un ensemble de données d'image, qui représente une image tridimensionnelle d'au moins une partie du corps (10) contenant l'objet (12), est fourni et la visibilité de l'objet (12) est déterminée à partir d'une image virtuelle qui est calculée à partir de l'ensemble de données d'image.

5. Procédé selon la revendication 4, **caractérisé en ce que** la position de l'objet (12) est déterminée à partir de l'ensemble de données d'image et des images des dispositifs d'imagerie (4, 5).

6. Procédé selon l'une des revendications 1 à 5, **caractérisé en ce qu'**un nombre d'alignements préférés est déterminé et ce nombre est affiché à un utilisateur pour sélectionner un ou plusieurs alignements.

7. Procédé selon l'une des revendications 1 à 5, **caractérisé en ce que** l'alignement optimal est automatiquement choisi parmi les alignements déterminés comme étant préférés.

8. Procédé selon l'une des revendications 1 à 7, **caractérisé en ce que** la visibilité de l'objet (12) est définie comme la partie de l'objet (12) visible dans une image par rapport à la taille globale de l'objet (12).

9. Programme informatique qui, lorsqu'il est exécuté dans une unité de calcul (6), permet à l'unité de calcul (6) de mettre en oeuvre le procédé selon l'une des revendications 1 à 8.

10. Dispositif pour déterminer des alignements préférés d'un générateur de faisceau de traitement (3) par rapport à un corps (10), lequel générateur de faisceau de traitement est adapté pour soumettre un objet à un rayonnement, **caractérisé par** une unité de calcul (6) qui est configurée pour tenir compte, lors de la détermination des alignements préférés, de la visibilité de l'objet (12) dans au moins une image, dans lequel l'image est obtenue au moyen d'au moins un dispositif d'imagerie (4, 5) dont la position relative par rapport au générateur de faisceau de traitement (3) est connue et invariable.

11. Dispositif de traitement (1) comportant un générateur de faisceau de traitement (3), au moins un dispositif d'imagerie (4, 5) dont la position relative par rapport au générateur de faisceau de traitement (3) est connue et invariable, et un dispositif selon la revendication 10.

12. Dispositif de traitement (1) selon la revendication 11, **caractérisé par** un support (2) sur lequel le générateur de faisceau de traitement (3) et les dispositifs d'imagerie (4, 5) sont agencés, et un mécanisme d'entraînement pour aligner le support (2) par rapport au corps (10).

13. Dispositif (1) selon l'une des revendications 11 à 12, **caractérisé en ce que** le dispositif d'imagerie (4, 5) est un dispositif à rayons X.

14. Dispositif (1) selon l'une des revendications 11 à 13, **caractérisé par** un dispositif d'affichage (13) pour afficher des alignements proposés ou sélectionnés.
